(19) Europäisches Patentamt
European Patent Office
Office européen des brevets

(11) EP 3 346 978 B1

(12) # EUROPEAN PATENT SPECIFICATION

(45) Date of publication and mention of the grant of the patent:
**14.06.2023 Bulletin 2023/24**

(21) Application number: **16709949.8**

(22) Date of filing: **15.03.2016**

(51) International Patent Classification (IPC):
***A61K 8/362*** *(2006.01)* ***A61K 8/44*** *(2006.01)*
***A61Q 5/04*** *(2006.01)* ***A61Q 5/10*** *(2006.01)*
***A61K 8/365*** *(2006.01)*

(52) Cooperative Patent Classification (CPC):
**A61Q 5/04; A61K 8/362; A61K 8/365; A61K 8/44; A61Q 5/10;** A61K 2800/805; A61K 2800/882; A61K 2800/884

(86) International application number:
**PCT/EP2016/055589**

(87) International publication number:
**WO 2017/041909 (16.03.2017 Gazette 2017/11)**

(54) **PROCESS FOR TREATING HAIR**

VERFAHREN ZUR HAARBEHANDLUNG

PROCÉDÉ DE TRAITEMENT DES CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.09.2015 PCT/EP2015/184305**

(43) Date of publication of application:
**18.07.2018 Bulletin 2018/29**

(73) Proprietor: **Kao Germany GmbH**
**64297 Darmstadt (DE)**

(72) Inventors:
- **NÖCKER, Bernd**
  **64297 Darmstadt (DE)**
- **BREAKSPEAR, Steven**
  **64297 Darmstadt (DE)**
- **BAUER, Peter**
  **64297 Darmstadt (DE)**
- **DÜRR, Manfred**
  **64297 Darmstadt (DE)**

(56) References cited:
**EP-A1- 1 655 056** **EP-A1- 2 020 254**
**EP-A1- 2 020 255** **US-A1- 2004 086 475**

- **Anonymous: "Washing Hair & Perm Service", Hairfinder , 15 May 2008 (2008-05-15), XP002752460, Retrieved from the Internet: URL:http://web.archive.org/web/20080515215701/http://www.hairfinder.com/hair2/permwashhair.htm [retrieved on 2015-12-18]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 346 978 B1

## Description

The present invention relates to a permanent shaping and dyeing process carried out one after another with compositions providing improved and milder shaping and dyeing of hair, especially human hair.

Hair dyeing and permanent shaping are carried out usually as two separate processes. Hair dyeing, especially under oxidative conditions, involves applying to hair a strongly oxidative composition comprising the dyestuff precursors and optionally coupling substances onto hair and leaving it for a certain period of time, usually at elevated temperatures, in order to allow for penetration of the relatively small uncolored dye precursors into the hair. In combination with the action of strong oxidizing agents, the dye precursors polymerize to larger molecules so that they may not be easily eluted from the hair. At the same time the effect of the oxidizing agent is to lighten the hair color to provide a relatively homogeneous dyeing base. Since the process involves the use of strong oxidative compositions, the hair fiber itself is negatively affected by such treatment and it consequently loses its certain natural cosmetic properties such as its strength against breaking, its natural elasticity, its natural shine and natural soft feel upon touching.

On the other hand, permanent shaping of hair involves application of a strongly reductive composition onto hair and leaving it for a certain period of time usually at elevated temperatures in order to open up the disulfide bonds and rebuilding them in the preferred shape with an application of a mild oxidative composition. Since the process involves the use of strong reductive and oxidative compositions, the hair fiber itself is affected by such treatment and therefore it also loses its certain natural properties such as its strength against breaking, its natural elasticity, its natural shine and natural soft feel upon touching.

Moreover, the to be dyed and/or permanently shaped hair is not always homogeneous in its physicochemical status as it may be damaged due to previous chemical treatments such as dyeing and permanently shaping and/or environmental effects. This often leads to inhomogeneous permanent shaping performance and/or inhomogeneous dyeing and, therefore, often consumers' dissatisfaction. There is, therefore, a great need for milder and more effective permanent shaping compositions which overcome one or more of the above mentioned problems.

There have been attempts to combine the two processes which have been successful to a certain extent. For example EP1655056, EP2020254, EP2020255, EP2191865 and EP2191864 disclose processes for permanent shaping and dyeing hair. However, these documents do not deal with the core of the present invention.

Recently in a series of patent applications (US2015/0034119, US2015/0037270, WO2015/017768) methods are published which claim benefits of the combined use of a bismaleate based binding agent in hair chemical treatments such as oxidative hair dyeing, permanently shaping and bleaching for improving hair structure. The publications are silent on the core of the present invention.

After a long research and careful considerations of the consumers' needs, the inventors of the present invention have unexpectedly found out that when commonly used permanent shaping compositions and dyeing compositions are mixed with another composition comprising predominantly carboxylic acids, the permanent shaping effect and dyeing effects are very much improved, homogeneous permanent shaping and dyeing of hair fibers is achieved and natural cosmetic properties of hair are maintained when the two processes are carried out one after another without the need of two separate hairdresser visits which saves time and certainly is more economical for the consumers.

Therefore the first object of the present invention is a process for treating hair, especially human hair, comprising the following steps:

a) hair is washed with a cleansing composition and towel dried,

b) hair is applied an aqueous composition (composition A) comprising one or more reducing agents, one or more alkalizing agents and has an alkaline pH in the range of 7.5 to 12.0, and is left on hair from 1 to 30 min,

c) hair is rinsed off with water

d) optionally hair is applied a composition C,
wherein the composition C is an aqueous composition comprising one or more oxidizing agents, preferably hydrogen peroxide, and has a pH in the range from 1.5 to 5,

e) hair is applied an aqueous composition consisting of two compositions B and C,

wherein B is an aqueous composition comprising one or more hair dyes and one or more alkalizing agents and has an alkaline pH preferably in the range of 7.1 to 12,

wherein the mixed composition has an alkaline pH and is obtained by mixing the compositions B and C immediately before application onto hair and left on the hair for a period of 1 to 45 min,

f) hair is rinsed off and optionally washed with a cleansing composition and dried,

wherein the composition(s) in step(s) b and/or e is (are) mixed with a composition D immediately before application onto hair for obtaining ready to use compositions,

wherein the composition D comprises

i) one or more carboxylic acids having three or more carboxyl groups and/or their salts selected from citric acid, ethylenediamine tetraacetic acid (EDTA), pyromellitic acid and glutamate diacetate, and
ii) one or more additional organic acid and/or their salts having two carboxyl groups selected from malic acid, tartaric acid, oxalic acid, malonic acid, succinic acid, glutaric acid, adipic acid, maleic acid, and fumaric acid,

wherein the composition D comprises the acids of i) and ii) and/or their salts at a total concentration of 10% to 100% by weight calculated to the total of composition D,

wherein the ready to use compositions have a pH in the range from 6.5 to 11 and comprise the acids and/or their salts at a total concentration in the range of 1 % to 10% by weight calculated to the total of the ready to use compositions,

wherein the hair is optionally put under tension before or during or after application of reducing composition in step b and the tension is released from hair before application of the composition in step e.

The second object is a kit for hair, especially human hair, comprising the compositions A, B, C and D.

The composition A comprises one or more reducing agents. Useful are thioglycolic acid and/or its salts, cysteamine and/or its salts, thioglycerin and/or its salts, glycerin esters of thioglycolic acid and/or its salts, thiolactic acid and/or its salts, cysteine or its derivatives and/or its salts and sodium sulfite. Preferred are thioglycolic acid and/or its salts, thiolactic acid and/or its salts and cysteine or its derivatives and/or its salts. The most preferred is thioglycolic acid and/or its salts.

One or more reducing agents may be comprised in the composition A at a concentration in the range of 1% to 15%, preferably 2% to 15%, more preferably 3% to 12.5% and most preferably 5% to 11 % by weight calculated to the total of composition A.

The composition A comprises one or more alkalizing agents. Suitable ones are ammonia and alkyl- or alkanolamines according to the general structure

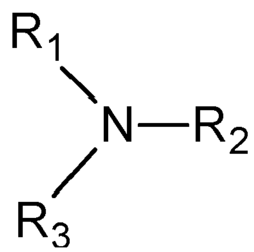

wherein $R_1$, $R_2$, and $R_3$ are same or different H, from $C_1$ to $C_4$, $C_3$ to $C_4$ unsaturated alkyl, $C_3$ to $C_4$ branched alkyl, $C_1$ to $C_4$ hydroxyl alkyl, $C_3$ to $C_4$ unsaturated hydroxyl alkyl, $C_3$ to $C_4$ branched hydroxyl alkyl, with the condition that at least one of $R_1$, $R_2$, or $R_3$ is different from H, wherein the alkalizing agents preferably selected from ammonia, monoethanolamine, and aminomethylpropanol, and particularly suitable one is aminomethylpropanol.

The alkalizing agent may be comprised in the composition A at a total concentration of 1% to 20%, preferably 1% to 17.5%, more preferably 2% to 15% and most preferably 2.5% to 13% by weight calculated to the total of the composition A.

The composition A has a pH in the range of 7.5 to 12, preferably 8 to 11, and more preferably 8.5 to 10.5 and most preferably 8 to 10 measured at 20°C.

The composition B comprises one or more hair dyes. Suitably, the composition B comprises one or more oxidative dye precursors and optionally one or more coupling substances.

Suitable non-limiting examples of oxidative dye precursor classes are p-phenylendiamines, p-aminophenols, and heterocyclic compounds such as diaminopyrazols and substituted pyrimidines, and suitable coupling substances are resorcinols, m-aminophenols, m-phenylendiamines, pyridines and its derivatives, and naphthols.

Non-limiting examples of the oxidative dye precursor compounds are p-phenylenediamine, p-aminophenol, 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene, 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxy pyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof, and mixture thereof.

The total concentration of the dye precursors (developing substances) customarily ranges between 0.001% to 5%, preferably 0.01% to 4% and more preferably 0.05% to 3%, and most preferably 0.1% to 2% by weight, calculated to the total of the composition B.

The suitable non-limiting examples of the coupling substance if present in the composition B are 5-amino-2-methylphenol, 2-methyl-5-hydroxyethylaminophenol, 2,4,-diaminophenoxyethanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, resorcinol, 2-methyl-resorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof and mixture thereof.

In the composition B the coupling substance(s) as reaction partners of the developing substance(s) may be present in approximately the same molecular proportions as the developing substances, i.e., at a total concentration in the range of 0.001% to 5%, preferably 0.01% to 4% and more preferably 0.05% to 3%, and most preferably 0.1% to 2% by weight, calculated to the total of the composition B.

Furthermore, the composition B may comprise one or more hair direct dyes. Suitable ones are cationic, anionic and nitro dyes. Plant dyes are also suitable for the compositions of the present invention.

Suitable anionic direct dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, HC Blue 18, HC Red 18 and HC Yellow 16 and their alkali metal salts such as sodium, potassium. Among those, the most preferred anionic dyestuffs are Acid Red 52, DC Violet 2, DC Red 33, DC Orange 4, DC Red 27, DC Yellow 10, HC Blue 18, HC Red 18 and HC Yellow 16.

Suitable cationic dyes are in principle those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87 Basic Orange 31 and HC Blue 17. The most preferred ones are Basic red 51, Basic Yellow 87 and Basic Orange 31 sold by BASF, and HC Blue 17.

Suitable nitro dyes are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1,

HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

The composition B may comprise one or more hair direct dye at a total concentration of 0.01% to 10%, preferably 0.05% to 7.5% and more preferably 0.1% to 5% by weight calculated to the total of the composition B. The composition can also comprise mixtures of several direct dyes, i.e., anionic, cationic and/or nonionic ones. In such a case the dyes may be mixed at any ratio with each other.

The composition B comprises furthermore one or more alkalizing agents. The suitable ones are same as the ones disclosed above for the composition A and preferred are ammonia, monoethanolamine, and aminomethylpropanol, and particularly suitable one is aminomethylpropanol.

The alkalizing agent may be comprised in the composition B at a total concentration of 1% to 20%, preferably 1% to 17.5%, more preferably 2% to 15% and most preferably 2.5% to 13% by weight calculated to the total of the composition B.

The pH of the composition B is preferably in the range of 7.1 to 12, more preferably 9 to 11, even more preferably 9 to 10.5 and most preferably 9.5 to 10.5.

The composition C is an aqueous composition and comprises one or more oxidizing agent(s). The oxidizing agents suitable are hydrogen peroxide, urea peroxide, melamin peroxide or perborate salts. The most preferred is hydrogen peroxide. The composition C may comprise one or more oxidizing agents at a total concentration of 0.75% to 20% by weight, preferably 1% to 15%, more preferably 2% to 12% and most preferably 3% to 12% by weight, calculated to the total of composition C. The composition C may be in the form of a solution, thickened gel or an emulsion. Emulsion form is particularly preferred.

The composition D comprises

i- one or more carboxylic acids having three or more carboxyl groups and/or their salts, and
ii- one or more additional organic acid and/or their salts having two carboxyl groups.

The carboxylic acids with three or more carboxyl groups and/or their salts are citric acid, ethylenediamine tetraacetic acid (EDTA), pyromellitic acid and glutamate diacetate. The ethylenediamine tetraacetic acid (EDTA) and/or its salts such as monosodium, disodium, trisodium and tetrasodium salts are the most preferred ones.

The organic acids with two carboxyl groups and/or their salts are malic acid, tartaric acid, oxalic acid, malonic acid, succinic acid, glutaric acid, adipic acid, maleic acid, and fumaric acid. The most preferred acid is malic acid and/or its salts such as sodium, potassium and ammonium salts.

The composition D comprises the two acids and/or their salts at a total concentration in the range of 10% to 100% by weight, preferably 12.5% to 90%, more preferably 12.5% to 75% by weight and most preferably 12.5% to 60% by weight, calculated to the total of composition D.

The two acids may be comprised in the composition D at a weight ratio of first acid (i) to second acid (ii) in the range from 10:1 to 1:250, preferably from 5:1 to 1:150, and more preferably from 2:1 to 1:100 and most preferably 1:50.

The composition D may be in the form of a powder, a dispersion, an emulsion or a solution. In a preferred embodiment of the present invention, the composition D is an aqueous composition and preferably has a pH in the range of 1 to 5, preferably 2 to 4, more preferably in the range of 2.5 to 3.6. In the case that the pH must be adjusted to a certain value, the composition D comprises one or more alkalizing agents as disclosed above for the composition A. The preferred are ammonia, monoethanolamine, and aminomethylpropanol and the aminomethylpropanol is particularly preferred.

The alkalizing agent may be comprised in the composition D at a total concentration of 1% to 20%, preferably 1% to 17.5%, more preferably 2% to 15% and most preferably 2.5% to 13% by weight calculated to the total of the composition D.

In a further preferred embodiment of the present invention, the composition D may comprise one or more thickening polymers selected from anionic, nonionic, cationic and amphoteric polymers, preferably selected from polymers with a viscosity of at least 500 mPa·s measured at a polymer concentration of 1% by weight in water and at 20°C with a Brookfield viscometer, such as at 10 rpm for 1 minute, with an appropriate spindle.

Suitable polymers are cellulose polymers, alginates, polysaccarides and acrylic acid polymers, preferably methyl cellulose, ethyl cellulose, hydroxyethylcellulose, methylhydroxyethylcellulose, methylhydroxypropylcellulose, carboxymethyl cellulose, alginic acids, sodium alginates, ammonium alginates, calcium alginates, gum arabic, guar gum

or xanthan gum, dehydroxanthan gum or acrylic acid polymers known with the CTFA adopted name Carbomer and its derivatives.

The preferred polymers are dehydroxanthan gum, xanthan gum, and polymeric anionic thickeners Carbomer and its derivatives. The particularly preferred thickening agent is dehydroxanthan gum. The thickening agents are preferably comprised in the composition D at a total concentration in the range of 0.1% to 5%, preferably, 0.2% to 3%, more preferably 0.25% to 2.5% and most preferably 0.3% to 2% by weight calculated to the total of the composition D.

The direct dyes disclosed above may also be comprised in the composition D at the same concentration ranges as disclosed above for the composition B.

The pH of the ready to use composition obtained by mixing the compositions A and D which is applied in step c of the process of present invention, is in the range of 6.5 to 11, preferably 7.5 to 10.5, more preferably 7.8 to 10 measured at 20°C.

The pH of the ready to use composition applied in step g which is obtained by mixing the compositions B, C and D is in the range of 6.5 to 11, preferably 6.8 to 10.5.

Any of the compositions A, B, C and/or D may comprise one or more of the commonly used hair conditioning compounds. These compounds are for example fatty alcohols, surfactants such as anionic, nonionic, cationic and amphoteric ones, ubiquinones, ceramides, organic solvents, lipophilic ingredients such as vegetable oils, mineral oils, silicones, fatty acid fatty alcohol esters, preservatives, amino acids, and polyols. It should be noted that these compounds are optionally comprised in the any of the compositions and their incompatibility must be carefully considered prior to addition in the compositions.

Any of the composition may comprise one or more fatty alcohols. In particular the compositions A and/or B may be aqueous compositions and may further be in the form of an emulsion and then comprise preferably one or more fatty alcohols. Suitable fatty alcohols are the ones with the chain length of 14 to 22 C atoms which may be saturated or unsaturated, linear or branched which may as well be substituted. Non-limiting examples are myristyl alcohol, cetyl alcohol, stearyl alcohol, behenyl alcohol and cetostearyl alcohol.

The total concentration of fatty alcohol may be in the range from 0.1% to 20%, preferably 0.5% to 15%, more preferably 1% to 10% by weight, calculated to the total of each composition.

Compositions A, B, C, and/or D according to the present invention may comprise surfactants selected from anionic, nonionic, amphoteric and/or cationic surfactants. The anionic, nonionic, amphoteric surfactants are used generally as emulsifier or solubilizer whereas the cationic surfactants are at the same time particularly used as hair conditioners.

Anionic surfactants suitable are in principle known from the cleansing compositions.

These are anionic surfactants of the sulfate, sulfonate, carboxylate and alkyl phosphate type, for example, the known $C_{10}$-$C_{18}$-alkyl sulfates, and in particular the respective ether sulfates, for example, $C_{12}$-$C_{14}$-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are $\alpha$-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids and salts thereof. Such products have been known for some time and are on the market, for example, under the trade name "AKYPO®" and "AKYPO-SOFT®".

Also useful are $C_8$-$C_{20}$-acyl isethionates, alone or in admixture with other anionic surfactants, as well as sulfofatty acids and the esters thereof.

Further suitable anionic surfactants are also $C_8$-$C_{22}$-acyl aminocarboxylic acids or the water-soluble salts thereof. Especially preferred is N-lauroyl glutamate, in particular as sodium salt, as well as, for example, N-lauroyl sarcosinate, N-$C_{12}$-$C_{18}$-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants.

Further surfactants suitable are nonionic surfactants. Non-limiting examples are long-chain fatty acid mono- and dialkanolamides, such as coco fatty acid mono- or diethanolamide and myristic fatty acid mono or diethanolamide, stearic acid mono or diethanolamide, alkyl polyglucosides with an alkyl group of 8 to 18 carbon atoms, and with 1 to 5 glucoside units, sorbitan esters, such as polyethylene glycol sorbitan stearic, myristic, palmitic, lauric acid esters, fatty acid polyglycol esters or polycondensates of ethyleneoxide and propyleneoxide, as they are on the market, for example, under the trade name "Pluronics$^R$", as well as fatty alcohol ethoxylates, $C_{10}$-$C_{22}$-fatty alcohol ethoxylates, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of

ethoxylation thereby ranges between about 2 and about 100, preferably about 10 and about 30.

Suitable amphoteric surfactants are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Suitable cationic surfactants are according to the general structure

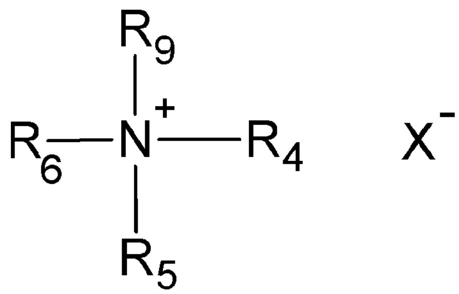

where $R_5$ is a saturated or unsaturated, branched or linear alkyl chain with 8-22 C atoms or

$R_7$ CO NH $(CH_2)_n$

where $R_7$ is saturated or unsaturated, branched or linear alkyl chain with 7-21 C atoms and n has typical value of 1 - 4 or

$R_8$ CO O $(CH_2)_n$

where $R_8$ is saturated or unsaturated, branched or linear alkyl chain with 7-21 C atoms and n has typical value of 1 - 4, and

$R_4$ is H or unsaturated or saturated, branched or linear alkyl chain with 1 - 22 C atoms or

$R_7$ CO NH $(CH_2)_n$

or

$R_8$ CO O $(CH_2)_n$

where $R_7$, $R_8$ and n are same as above.

$R_9$ and $R_6$ are H or lower alkyl chain with 1 to 4 Carbon atoms, and X is typically chloride, bromide, methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Concentration of one or more total surfactants in any of the compositions A, B, C and/or D may be in the range of 0.1% to 20%, preferably 0.2% to 15% and most preferably 0.2% to 10% by weight, calculated to the total of each composition.

The compositions A, B, C and/or D may further comprise lipophilic ingredients such as vegetable oils, for example, jojoba oil or any other; liquid paraffins, especially paraffinum perliquidum and parafiinum subliquidum; silicones for example linear polysiloxanes such as dimethicones with various consistency and dimethiconols, aminated silicones with primary, secondary, tertiary or quaternary ammonium groups such as amodimethicone, polysilicone 9, and quaternium 80, cyclic silicones such as cyclomethicones, arylated silicones such as phenyl trimethicone; fatty acid esters such as octyl palmitate, isocetyl palmitate, isopropyl palmitate and octyl stearate, $C_{10}$-to $C_{36}$-fatty acid triglycerides, as well as their mixtures. Total concentration of these lipophilic compounds may be in the range of 0.1% to 20% by weight, preferably from 1% to 15% by weight, and more preferably from 2% to 10% by weight, calculated to the total of each composition.

Compositions A, B, C and/or D can also comprise cationic polymers as conditioning and/or thickening agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 4, Polyquaternium 6, Polyquaternium 7, Polyquaternium 10, Polyquaternium 11, Polyquaternium 16, Polyquaternium 22, Polyquaternium 24, Polyquaternium 28, Polyquaternium 30, Polyquaternium 37, Polyquaternium 36, Polyquaternium 46, Polyquaternium 67, and Polyquaternium 72.

Equally suitable are those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

The total concentration of cationic polymers may be in the range of 0.1% to 7.5% by weight, preferably 0.3% to 5% by weight and more preferably 0.5% to 2.5% by weight, calculated to the total of each composition

Composition A, B, C and/or D may comprise one or more ceramide compound, such as the one according to general formula

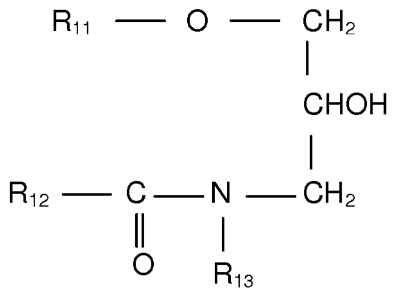

where $R_{11}$ and $R_{12}$ are independent from each other an alkyl- or alkenyl group with 10 to 22 carbon atoms, $R_{13}$ is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide. Concentration of ceramide type of compounds ranges from 0.01% to 2%, preferably 0.01% to 1% by weight calculated to the total of each composition.

The compositions A and/or B may comprise ubiquinone of the formula:

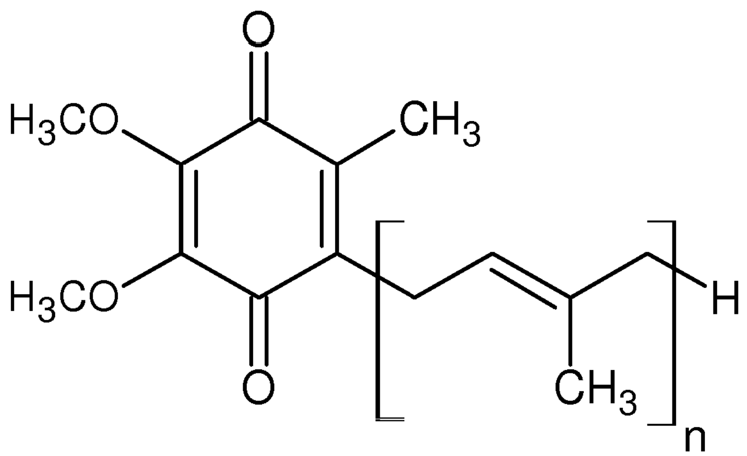

wherein n is a number from 1 to 10. Concentration of ubiquinone can vary between 0.001% and 10% by weight, calculated to the total of each composition.

The compositions A, B, C and/or D may comprise one or more organic solvent such as 2-phenoxyethanol, benzyl alcohol, 2-phenylethanol and 2-benzyloxyethanol. Suitable aliphatic alcohols are ethanol, isopropanol, propanol, n-butanol, isobutanol, t-butanol and 1-pentanol. Concentration of one or more organic solvent may be in the range of 0.1% to 15%, preferably 0.5% to 12.5% and more preferably 1% to 10% and most preferably 1% to 7.5% by weight calculated to the total of each composition.

The compositions A, B, C and/or D may further comprise one or more amino acids, preferably at a concentration in the range of 0.01% to 5%, preferably 0.1% to 3% and more preferably 0.2% to 2.5% and most preferably 0.25% to 2% by weight calculated to the total of each composition. Suitable ones are all of the known amino acids such as, arginine. alanine, asparagine, glutamine, glycine, histidine, leucine, lysine, methionine, phenylalanine, proline, serine, threonine, tryptophan, tyrosine and valine.

The compositions A, B, C and/or D may further comprise one or more polyol, preferably at a concentration in

the range of 0.01% to 5%, preferably 0.1% to 3% and more preferably 0.2% to 2.5% and most preferably 0.25% to 2% by weight calculate to the total of each composition. Suitable ones are propylene glycol, diproplylene glycol, glycerine, panthenol and its derivatives.

The compositions A, B, C and/or D may further comprise any known preservatives if necessary.

After rinsing off the reducing composition in step c and prior to application of the composition C and or prior to releasing the tension from hair, an aqueous intermediate treatment composition may preferably be used in order to de-swell hair for minimizing further damage to the hair fibre. The intermediate composition may be applied onto hair after rinsing off the reducing composition but before applying the oxidizing composition and preferably left on the hair up to 15 min, more preferably up to 10 min and optionally rinsed off from hair prior to application of the oxidizing composition in steps d or e. The intermediate composition comprises one or more inorganic salt, preferably at a concentration of 0.5% to 15%, more preferably 1% to 12.5% and most preferably 2% to 12.5% by weight calculated to the total of the composition.

In principle any water soluble inorganic salt is suitable for the purpose. In the preferred embodiment, salts are preferably selected from salts of mono or divalent cations with mono and divalent anions. Preferred cations are sodium, calcium, potassium and magnesium and anions are chloride and sulfate. Suitable ones are such as sodium chloride, sodium sulfate, magnesium sulfate, potassium chloride, potassium sulfate, magnesium chloride, calcium chloride, ammonium salts such as ammonium chloride and ammonium sulfate. Additionally salts have been found to be especially suitable such as iodide ions especially potassium and sodium salts, copper chloride, copper sulphate, cobalt chloride, cerium sulphate, cerium chloride, vanadium sulphate, lithium chloride, magnesium acetate, calcium nitrate, barium nitrate, magnesium oxide, and ammonium nitrate. Preferred inorganic salts are sodium chloride, sodium sulfate, magnesium sulfate, potassium chloride, potassium sulfate, magnesium chloride, calcium chloride and salts of iodide ions. More preferably the salts are sodium chloride, sodium sulfate, magnesium sulfate, potassium chloride, potassium sulfate, magnesium chloride and salts of iodide ions especially potassium and sodium salts. In particular, with magnesium sulfate, sodium chloride and potassium iodide exceptionally good results are observed.

The intermediate treatment composition may preferably comprise an oxidizing agent at a concentration of 0.1% to 5%, preferably 0.2% to 5% more preferably 0.2% to 3% and most preferably 0.2% to 2% by weight calculated to the total of the composition. Suitable oxidizing agents are hydrogen peroxide and sodium bromate. Most preferred is hydrogen peroxide.

The intermediate treatment composition has a pH between 2 and 7, preferably 2.5 and 6 and more preferably 3 and 5.

The following examples are to illustrate the invention.

## Example 1

### The composition A

| | % by weight |
|---|---|
| Ammonium thioglycolate (60%) | 21.3 |
| Ammonium hydrogen carbonate | 5.0 |
| 1,3-butylene glycol | 3.0 |
| Amodimethicone | 0.2 |
| PEG-40-Hydrogenated castor oil | 0.7 |
| Fragrance | 0.4 |
| Ammonia, 25% | ad pH 8.3 |
| Water | ad 100.0 |

### The composition B

| | % by weight |
|---|---|
| Cetearyl alcohol | 10.0 |
| Cocamide MEA | 4.0 |
| Sodium lauryl sulphate | 1.5 |

(continued)

| | % by weight |
|---|---|
| Propylene glycol | 2.0 |
| Cetyltrimonium chloride | 0.5 |
| 2,5,6-Triamino-4-hydroxypyrimidine sulphate | 0.01 |
| 2,5-Diaminotoluene sulphate | 0.55 |
| 4-Chlororesorcinol | 0.17 |
| Resorcinol | 0.05 |
| 3-Aminophenol | 0.03 |
| Sodium sulfite | 1.0 |
| Aminomethylpropanol | 2.0 |
| Ammonium hydroxide | q.s. to pH 10.0 |
| Fragrance, preservative | q.s. |
| Water | to 100 |

**The Composition C**

| | % by weight |
|---|---|
| Hydrogen peroxide | 2 |
| Phosphoric acid | q.s. to pH 3.5 |
| Water | to 100 |

**The composition D**

| | % by weight |
|---|---|
| Tetrasodium EDTA | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition D is approximately 3.5.

Caucasian hair of 25 cm length was obtained from Fischbach + Miller, Laupheim, Germany. The hair streaks were permanently straightened and dyed with the above compositions. First, the hair was shampooed with a Dualsenses Deep Cleansing Shampoo, towel dried and put on curlers and the reducing composition (the composition A) given above was applied onto hair after mixing with the composition D at a weight ratio of composition A to D 10:0.2 and processed for about 15 minutes. Then the composition was rinsed off from the hair and the dyeing composition obtained by mixing the compositions B, C and D at a weight ratio of 1 :2:0.2 was applied onto hair. The resulting composition had a pH of approximately 9.5. The composition was applied onto streaks of human hair and left on the hair for 30 min at ambient temperature, then rinsed off from the hair and the hair was shampooed with a Dualsenses Color Protection Shampoo and dried (inventive process).

For comparison purposes a similar process of above was applied to hair with the exception that composition D was replaced with an equal amount of water instead of composition D (comparative process).

Evenness of perm and color durability were investigated on pre-damaged hair. Damage was conferred to hair by bleaching hair with a commercially available bleaching composition under the brand Goldwell. Then, the inventive and comparative process of above was applied to hair. The result was recorded by measuring spreading of the hair streaks and calculation of a volume factor based on the spreading of hair at the root and at the tip parts. In other words,

width of hair streaks were measured at their root and at their tips by placing the hair streaks on millimeter paper. A volume factor was calculated according to equation (1):

$$Volumefactor = \frac{Width\ at\ hair\ tips\ [cm]}{Width\ at\ hair\ root\ [cm]} \qquad \text{Equation (1)}$$

Hair streaks before treatment displayed a volume factor of 1.2. As a result of the experiments, the hair treated with the inventive process had a volume factor of 1.45, whereas the hair treated with the comparative process had a volume factor of 2.63. In conclusion, the comparative process led to a much higher increase of hair volume which is definitively undesired by the customer as a result of a straightening process. The inventive process did not lead to such an increase.

Color durability was investigated by incubating the hair streaks in a shaking bath with a shaking frequency of 100 $min^{-1}$ for 15 min at 30°C. The water bath was filled with an aqueous solution of sodium laureth sulfate at a concentration of 5% by weight, calculated to the total of the water bath solution. Upon incubation, the hair streaks were rinsed with water and towel dried. Color results were measured prior to incubation and upon incubation by spectrophotometrical analysis with a Datacolor 45G CT instrument delivered from Datacolor Inc., Lawrenceville, NJ, USA. Based on the CIE*Lab color space results obtained by the measurements, $\Delta E_{ab}$ values for color difference were calculated according to equation (1):

$$\Delta E_{ab} = \sqrt{(L_2 - L_1) + (a_2 - a_1) + (b_2 - b_1)} \qquad \text{Equation 1}$$

The $\Delta E_{ab}$ value for hair streaks treated with the inventive process was 10.93, whereas the $\Delta E_{ab}$ value for the hair streaks treated with the comparative process was 13.75. Lower $\Delta E_{ab}$ values correspond to less change in hair color upon incubation and the results clearly showed that the inventive process led to a much lower color change which differs to the comparative process by approximately 3 color units. In conclusion the presented data clearly showed the superior performance of the inventive process compared to the state-of-the-art process.

## Example 2

### Intermediate composition

| | % by weight |
|---|---|
| Magnesium sulfate | 10 |
| Cetrimonium chloride | 0.5 |
| Citric acid | q.s. to pH 4.2 |
| Water | q.s. to 100 |

In the process disclosed with Example 1 above, the intermediate treatment composition was applied onto hair after rinsing off the reducing composition. The intermediate treatment composition was left on hair for 5 min and without rinsing it off the composition of step e was applied. The permanent shaping and dyeing results observed with the Example 1 were confirmed.

Similar results were obtained with the following compositions.

## Example 3

### The composition D

| | % by weight |
|---|---|
| Tetrasodium EDTA | 5.0 |
| Malic acid | 15.0 |

(continued)

| | % by weight |
|---|---|
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition D was approximately 3.6.

**Example 4**

**The composition D**

| Component | % by weight |
|---|---|
| AMP | 6.0 |
| Tetrasodium EDTA | 3.0 |
| Malic acid | 13.0 |
| Lactic acid | 4.0 |
| Hydroxypropyl xanthan gum | 0.6 |
| Polyquaternium-10 | 0.1 |
| Water | to 100 |
| pH | 3.4±0.1 |

**Example 5:**

**The composition D**

| Component | % by weight |
|---|---|
| Monoethanolamine (MEA) | 2.7 |
| Tetrasodium EDTA | 5.0 |
| Malic acid | 15.0 |
| Hydroxypropyl xanthan gum | 0.6 |
| Panthenol | 0.1 |
| Water | To 100 |
| pH | 3.3±0.1 |

**Example 6:**

**The composition D**

| Component | % by weight |
|---|---|
| AMP | 6.0 |
| Citric acid | 5.0 |
| Maleic acid | 15.0 |
| Hydroxypropyl xanthan gum | 0.6 |
| Behenamidopropyl trimonium chloride | 0.2 |
| Water | to 100 |
| pH | 1.5±0.1 |

**Example 7:**

**The composition D** (not according to the invention as claimed)

| Component | % by weight |
|---|---|
| MEA | 2.0 |
| Lactic acid | 15.0 |
| Citric acid | 6.0 |
| Hydroxypropyl xanthan gum | 0.6 |
| Polyquaternium-67 | 0.1 |
| Water | to 100 |
| pH | 2.7±0.1 |

**Example 8**

**The composition D**

| | % by weight |
|---|---|
| Tetrasodium EDTA | 1.0 |
| Malic acid | 13.0 |
| Aminomethylpropanol | 6.0 |
| Basic red 51 | 1.00 |
| HC red XX | 1.00 |
| Hydroxypropyl xanthan gum | 0.6 |
| Cetrimonium chloride | 0.1 |
| Preservative | q.s. |
| Water | to 100 |

The pH of the composition was 3.5.

**Example 9**

**The composition A**

| | % by weight |
|---|---|
| Ammonium thioglycolate (60%) | 0.9 |
| Cystein hydrochloride | 5.7 |
| Ammonium hydrogen carbonate | 1.5 |
| Acetylcystein | 0.7 |
| Cetrimonium chloride | 0.1 |
| 1,3-butylene gylcol | 0.5 |
| Amodimethicone | 0.2 |
| Fragrance | 0.4 |
| Ammonia, 25% | ad pH 9.8 |
| Water | q.s. 100.0 |

The composition B and C were same as Example 1.

**The composition D**

| | % by weight |
|---|---|
| Tetrasodium EDTA | 4.0 |
| Malic acid | 17.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the composition was 3.4.

**Example 10**

**The composition D (powder)**

| | % by weight |
|---|---|
| Tetrasodium EDTA | 7.0 |
| Malic acid | 92.0 |
| Hydroxyethylcellulose | 1.0 |

1 g of the composition above was added to the mixture of 30 g of composition A of example 1. After mixing thoroughly, the resulting composition was applied onto hair which was already put under tension using curlers and rinsed off after leaving it on the hair for 30 min. The hair was released from tension and the dyeing composition obtained by mixing the compositions B (20 g), C (20 g) and D (1g) was applied onto hair and after leaving on the hair for 30 min rinsed off from hair and hair was shampooed and dried. It was observed that the hair was effectively and homogeneously curled and dyed and had its natural softness and elasticity.

**Example 11**

**The composition D**

| | % by weight |
|---|---|
| EDTA monosodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition D is approximately 3.1.

**Example 12**

**The composition D**

| | % by weight |
|---|---|
| EDTA disodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition D is approximately 3.2.

**Example 13**

**The composition D**

| | % by weight |
|---|---|
| EDTA trisodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition D is approximately 3.4.

## Claims

1. **1.** Process for treating hair, especially human hair, comprising the following steps:

   a) hair is washed with a cleansing composition and towel dried,
   b) hair is applied an aqueous composition (composition A) comprising one or more reducing agents, one or more alkalizing agents and has an alkaline pH in the range of 7.5 to 12.0, and is left on hair from 1 to 30 min,
   c) hair is rinsed off with water
   d) optionally hair is applied a composition C,
   wherein the composition C is an aqueous composition comprising one or more oxidizing agents, preferably hydrogen peroxide, and has a pH in the range from 1.5 to 5,
   e) Hair is applied an aqueous composition consisting of two compositions B and C,

   wherein B is an aqueous composition comprising one or more hair dyes and one or more alkalizing agents and has an alkaline pH preferably in the range of 7.1 to 12,
   wherein the mixed composition has an alkaline pH and is obtained by mixing the compositions B and C immediately before application onto hair and left on the hair for a period of 1 to 45 min,

   f) Hair is rinsed off and optionally washed with a cleansing composition and dried,

   wherein the composition(s) in step(s) b) and/or e) is (are) mixed with a composition D immediately before application onto hair for obtaining ready to use compositions,
   wherein the composition D comprises

   i) one or more carboxylic acids having three or more carboxyl groups and/or their salts selected from citric acid, ethylenediamine tetraacetic acid (EDTA), pyromellitic acid and glutamate diacetate, and
   ii) one or more additional organic acid and/or their salts having two carboxyl groups selected from the group of malic acid, tartaric acid, oxalic acid, malonic acid, succinic acid, glutaric acid, adipic acid, maleic acid, and fumaric acid,

   wherein the composition D comprises the acids of i) and ii) and/or their salts at a total concentration of 10% to 100% by weight calculated to the total of composition D,
   wherein the ready to use compositions have a pH in the range from 6.5 to 11 and comprise the acids and/or their salts at a total concentration in the range of 1% to 10% by weight calculated to the total of the ready to use compositions,
   wherein the hair is optionally put under tension before or during or after application of reducing composition in step b and the tension is released from hair before application of the composition in step e.

2. **2.** The process according to claim 1 wherein the composition D comprises the first acid (i) and the second acid (ii) at a weight ratio (i)/(ii) in the range from 10:1 to 1:250, preferably from 5:1 to 1:150, and more preferably from 2:1 to 1:100 and most preferably 1:50.

3. **3.** The process according to claims 1 to 2 wherein the composition D is a powder, a dispersion, an emulsion or a solution, preferably it is an aqueous composition.

4. **4.** The process according to any of the preceding claims **characterized in that** the pH of the composition D ranges from 1 to 5, and composition D comprises an alkalizing agent.

5. **5.** The process according to any of the proceeding claims wherein the carboxylic acid with three or more carboxyl groups is EDTA and/or its salts.

6. **6.** The process according to any of the proceeding claims wherein the organic acid with two carboxyl groups is malic acid and/or its salts.

7. **7.** The process according to any of the proceeding claims wherein the composition B comprises one or more oxidative dye precursors, preferably selected from p-phenylendiamines, p-aminophenols, and heterocyclic diamines such as diaminepyrazols, and optionally one or more coupling substances preferably selected from resorcinols, m-aminophenols, m-phenylendiamines, pyridines and its derivatives, and naphthols.

**8.** The process according to any of the proceeding claims wherein the composition(s) B and/or D comprise(s) one or more hair direct dye selected from cationic, anionic, and nitro dyes or their mixtures.

**9.** The process according to any of the preceding claims wherein the composition(s) A and/or B and/or D comprise(s) at least one alkalizing agent selected from ammonia, alkyl- or alkanolamines according to the general structure

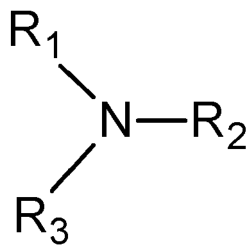

wherein $R_1$, $R_2$, and $R_3$ are same or different H, from $C_1$ to $C_4$, $C_3$ to $C_4$ unsaturated alkyl, $C_3$ to $C_4$ branched alkyl, $C_1$ to $C_4$ hydroxyl alkyl, $C_3$ to $C_4$ unsaturated hydroxyl alkyl, $C_3$ to $C_4$ branched hydroxyl alkyl, with the condition that at least one of $R_1$, $R_2$, or $R_3$ is different from H, wherein the alkalizing agents preferably selected from ammonia, monoethanolamine, and aminomethylpropanol, and particularly suitable one is aminomethylpropanol.

**10.** The process according to any of the preceding claims wherein the composition A, B, C, and/or D comprise one or more ingredients, selected from fatty alcohols, surfactants selected from anionic, nonionic, cationic and amphoteric ones, ubiquinones, ceramides, reducing agents, organic solvents, silicones such as linear polysiloxanes, aminated silicones, cyclic silicones, arylated silicones, antioxidants, preservatives, amino acids, polyols.

**11.** The process according to any of the preceding claims wherein the composition A and/or B comprise reducing agents selected from thioglycolic acid, cysteamine and/or its salts, thioglycerin and/or its salts, glycerin esters of thioglycolic acid and/or its salts, thiolactic acid and/or its salts, cysteine or its derivatives and/or its salts, wherein the composition A preferably comprises the reducing agents at a concentration range from 1% to 15% by weight calculated to the total of composition A, and wherein the composition B preferably comprises the reducing agents at a concentration range from 0.1% to 1.0% by weight calculated to the total of composition B.

**12.** The process according to any of the preceding claims wherein the composition D comprises one or more thickening polymers selected from anionic, nonionic, cationic and amphoteric polymers, preferably selected from polymers having a viscosity of at least 500 mPa·s measured at a polymer concentration of 1% by weight in water and at 20°C with a Brookfield viscometer, such as at 10 rpm for 1 minute, with an appropriate spindle.

**13.** The process according to claim 12 wherein the thickening polymer is selected from hydroxypropyl xanthan gum, dehydroxanthan gum, xanthan gum, and polymeric anionic thickeners.

**14.** The process according to any of the preceding claims wherein the alkalizing agent is comprised in the composition A, B, and/or D at a concentration in the range of 0.1% to 20% by weight calculated to the total of each composition.

**15.** Kit for hair comprising the compositions A, B, C and D as described in the claims 1 to 14.

**Patentansprüche**

**1.** Verfahren zur Behandlung von Haaren, insbesondere menschlichen Haaren, umfassend die folgenden Schritte:

a) Das Haar wird mit einem Reinigungsmittel gewaschen und mit einem Handtuch getrocknet,
b) auf die Haare wird eine wässrige Zusammensetzung (Zusammensetzung A) aufgetragen, die ein oder mehrere Reduktionsmittel und ein oder mehrere Alkalisierungsmittel enthält und einen alkalischen pH-Wert im Bereich von 7,5 bis 12,0 aufweist, und 1 bis 30 min auf den Haaren belassen wird,
c) die Haare werden mit Wasser ausgespült,
d) gegebenenfalls wird auf das Haar eine Zusammensetzung C aufgetragen,
wobei die Zusammensetzung C eine wässrige Zusammensetzung ist, die ein oder mehrere Oxidationsmittel, vorzugsweise Wasserstoffperoxid, enthält und einen pH-Wert im Bereich von 1,5 bis 5 aufweist,

e) Auf das Haar wird eine wässrige Zusammensetzung aufgetragen, die aus zwei Zusammensetzungen B und C besteht,

worin B eine wäßrige Zusammensetzung ist, die einen oder mehrere Haarfarbstoffe und ein oder mehrere Alkalisierungsmittel enthält und einen alkalischen pH-Wert vorzugsweise im Bereich von 7,1 bis 12 aufweist, wobei die gemischte Zusammensetzung einen alkalischen pH-Wert aufweist und durch Mischen der Zusammensetzungen B und C unmittelbar vor dem Auftragen auf das Haar und Einwirkenlassen auf das Haar für einen Zeitraum von 1 bis 45 Minuten erhalten wird,

f) Das Haar wird ausgespült und gegebenenfalls mit einer Reinigungszusammensetzung gewaschen und getrocknet,

wobei die Zusammensetzung(en) aus Schritt(en) b und/oder e mit einer Zusammensetzung D unmittelbar vor der Anwendung auf dem Haar gemischt wird (werden), um gebrauchsfertige Zusammensetzungen zu erhalten,
wobei die Zusammensetzung D umfasst

i) eine oder mehrere Carbonsäuren mit drei oder mehr Carboxylgruppen und/oder ihre Salze, ausgewählt aus Zitronensäure, Ethylendiamintetraessigsäure (EDTA), Pyromellitsäure und Glutamatdiacetat, und
ii) eine oder mehrere zusätzliche organische Säuren und/oder ihre Salze mit zwei Carboxylgruppen, ausgewählt aus der Gruppe von Äpfelsäure, Weinsäure, Oxalsäure, Malonsäure, Bernsteinsäure, Glutarsäure, Adipinsäure, Maleinsäure und Fumarsäure,

wobei die Zusammensetzung D die Säuren von i) und ii) und/oder ihre Salze in einer Gesamtkonzentration von 10 bis 100 Gew.-%, berechnet auf die Gesamtmenge der Zusammensetzung D, enthält,
wobei die gebrauchsfertigen Zusammensetzungen einen pH-Wert im Bereich von 6,5 bis 11 aufweisen und die Säuren und/oder ihre Salze in einer Gesamtkonzentration im Bereich von 1 bis 10 Gew.-%, berechnet auf die Gesamtmenge der gebrauchsfertigen Zusammensetzungen, enthalten,
wobei das Haar gegebenenfalls vor oder während oder nach dem Auftragen der reduzierenden Zusammensetzung in Schritt b unter Spannung gesetzt wird und die Spannung vor dem Auftragen der Zusammensetzung in Schritt e vom Haar gelöst wird.

**2.** Verfahren nach Anspruch 1, wobei die Zusammensetzung D die erste Säure (i) und die zweite Säure (ii) in einem Gewichtsverhältnis (i)/(ii) im Bereich von 10:1 bis 1:250, vorzugsweise von 5:1 bis 1:150 und noch bevorzugter von 2:1 bis 1:100 und am meisten bevorzugt 1:50 umfasst.

**3.** Verfahren nach einem der Ansprüche 1 bis 2, wobei die Zusammensetzung D ein Pulver, eine Dispersion, eine Emulsion oder eine Lösung ist, vorzugsweise eine wässrige Zusammensetzung.

**4.** Verfahren nach einem der vorhergehenden Ansprüche, **dadurch gekennzeichnet, dass** der pH-Wert der Zusammensetzung D im Bereich von 1 bis 5 liegt und die Zusammensetzung D ein Alkalisierungsmittel enthält.

**5.** Verfahren nach einem der vorangehenden Ansprüche, wobei die Carbonsäure mit drei oder mehr Carboxylgruppen EDTA und/oder ihre Salze ist.

**6.** Verfahren nach einem der vorangehenden Ansprüche, wobei die organische Säure mit zwei Carboxylgruppen Apfelsäure und/oder deren Salze ist.

**7.** Verfahren nach einem der vorangehenden Ansprüche, wobei die Zusammensetzung B einen oder mehrere oxidative Farbstoffvorläufer, vorzugsweise ausgewählt aus p-Phenylendiaminen, p-Aminophenolen und heterocyclischen Diaminen, wie Diaminpyrazolen, und gegebenenfalls eine oder mehrere Kupplungssubstanzen, vorzugsweise ausgewählt aus Resorcinolen, m-Aminophenolen, m-Phenylendiaminen, Pyridinen und ihren Derivaten und Naphtholen, enthält.

**8.** Verfahren nach einem der vorangehenden Ansprüche, wobei die Zusammensetzung(en) B und/oder D einen oder mehrere Haardirektfarbstoffe, ausgewählt aus kationischen, anionischen und Nitrofarbstoffen oder deren Mischungen, enthält/enthalten.

9. Verfahren nach einem der vorhergehenden Ansprüche, wobei die Zusammensetzung(en) A und/oder B und/oder D mindestens ein Alkalisierungsmittel, ausgewählt aus Ammoniak, Alkyl- oder Alkanolaminen, gemäß der allgemeinen Struktur

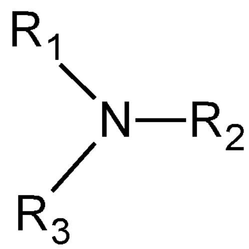

worin $R_1$, $R_2$ und $R_3$ gleich oder verschieden sind von H, von $C_1$ bis $C_4$, $C_3$ bis $C_4$ ungesättigtem Alkyl, verzweigtem $C_3$ bis $C_4$ Alkyl, $C_1$ bis $C_4$ Hydroxylalkyl, ungesättigtem $C_3$ bis $C_4$ Hydroxylalkyl, verzweigtem $C_3$ bis $C_4$ Hydroxylalkyl, mit der Bedingung, dass mindestens eines von $R_1$, $R_2$ oder $R_3$ verschieden ist von H, wobei die Alkalisierungsmittel vorzugsweise ausgewählt sind aus Ammoniak, Monoethanolamin und Aminomethylpropanol, und besonders geeignet ist Aminomethylpropanol.

10. Verfahren nach einem der vorhergehenden Ansprüche, wobei die Zusammensetzung A, B, C und/oder D einen oder mehrere Bestandteile umfasst, die ausgewählt sind aus Fettalkoholen, Tensiden, die aus anionischen, nichtionischen, kationischen und amphoteren Tensiden ausgewählt sind, Ubichinonen, Ceramiden, Reduktionsmitteln, organischen Lösungsmitteln, Silikonen wie linearen Polysiloxanen, aminierten Silikonen, cyclischen Silikonen, arylierten Silikonen, Antioxidantien, Konservierungsmitteln, Aminosäuren, Polyolen.

11. Verfahren nach einem der vorhergehenden Ansprüche, wobei die Zusammensetzung A und/oder B Reduktionsmittel enthält, die ausgewählt sind aus Thioglykolsäure, Cysteamin und/oder seinen Salzen, Thioglycerin und/oder seinen Salzen, Glycerinestern der Thioglykolsäure und/oder seinen Salzen, Thiomilchsäure und/oder seinen Salzen, Cystein oder dessen Derivate und/oder dessen Salze, wobei die Zusammensetzung A vorzugsweise die Reduktionsmittel in einem Konzentrationsbereich von 1 bis 15 Gew.-%, berechnet auf die Gesamtmenge der Zusammensetzung A, enthält, und wobei die Zusammensetzung B vorzugsweise die Reduktionsmittel in einem Konzentrationsbereich von 0. 1 Gew.-% bis 1,0 Gew.-%, berechnet auf das Gesamtgewicht der Zusammensetzung B, enthält.

12. Verfahren nach einem der vorhergehenden Ansprüche, wobei die Zusammensetzung D ein oder mehrere verdickende Polymere, ausgewählt aus anionischen, nichtionischen, kationischen und amphoteren Polymeren, umfaßt, vorzugsweise ausgewählt aus Polymeren mit einer Viskosität von mindestens 500 mPa-s, gemessen bei einer Polymerkonzentration von 1 Gew.-% in Wasser und bei 20°C mit einem Brookfield-Viskosimeter, beispielsweise bei 10 U/min für 1 Minute, mit einer geeigneten Spindel.

13. Verfahren nach Anspruch 12, wobei das verdickende Polymer ausgewählt ist aus Hydroxypropyl-Xanthan-Gummi, Dehydroxanthan-Gummi, Xanthan-Gummi und polymeren anionischen Verdickungsmitteln.

14. Verfahren nach einem der vorangehenden Ansprüche, wobei das Alkalisierungsmittel in der Zusammensetzung A, B und/oder D in einer Konzentration im Bereich von 0,1 bis 20 Gew.-%, berechnet auf die Gesamtmenge jeder Zusammensetzung, enthalten ist.

15. Kit für das Haar, umfassend die Zusammensetzungen A, B, C und D gemäß den Ansprüchen 1 bis 14.

## Revendications

1. Procédé de traitement des cheveux, en particulier des cheveux humains, comprenant les étapes suivantes :

   a) les cheveux sont lavés avec une composition nettoyante et séchés à la serviette,
   b) on applique sur les cheveux une composition aqueuse (composition A) comprenant un ou plusieurs agents réducteurs, un ou plusieurs agents alcalinisants et ayant un pH alcalin compris entre 7,5 et 12,0, que l'on laisse agir de 1 à 30 minutes,
   c) les cheveux sont rincés à l'eau,

d) les cheveux sont éventuellement recouverts d'une composition C,
dans laquelle la composition C est une composition aqueuse comprenant un ou plusieurs agents oxydants, de préférence du peroxyde d'hydrogène, et dont le pH est compris entre 1,5 et 5,
e) Les cheveux sont soumis à une composition aqueuse constituée de deux compositions B et C,

dans laquelle B est une composition aqueuse comprenant un ou plusieurs colorants capillaires et un ou plusieurs agents alcalinisants et dont le pH alcalin est de préférence compris entre 7,1 et 12,
la composition mélangée a un pH alcalin et est obtenue en mélangeant les compositions B et C immédiatement avant l'application sur les cheveux et en les laissant sur les cheveux pendant une période de 1 à 45 minutes,

f) Les cheveux sont rincés et éventuellement lavés avec une composition nettoyante et séchés,

dans laquelle la ou les compositions des étapes b et/ou e sont mélangées avec une composition D immédiatement avant l'application sur les cheveux pour obtenir des compositions prêtes à l'emploi,
dans laquelle la composition D comprend

i) un ou plusieurs acides carboxyliques ayant trois groupes carboxyles ou plus et/ou leurs sels choisis parmi l'acide citrique, l'acide éthylènediamine tétraacétique (EDTA), l'acide pyromellitique et le diacétate de glutamate, et
ii) un ou plusieurs autres acides organiques et/ou leurs sels ayant deux groupes carboxyle choisis dans le groupe de l'acide malique, de l'acide tartrique, de l'acide oxalique, de l'acide malonique, de l'acide succinique, de l'acide glutarique, de l'acide adipique, de l'acide maléique et de l'acide fumarique,

dans laquelle la composition D comprend les acides i) et ii) et/ou leurs sels à une concentration totale de 10 % à 100 % en poids calculée par rapport au total de la composition D,
les compositions prêtes à l'emploi ont un pH compris entre 6,5 et 11 et comprennent les acides et/ou leurs sels à une concentration totale comprise entre 1 % et 10 % en poids calculé par rapport au total des compositions prêtes à l'emploi,
les cheveux sont éventuellement mis sous tension avant, pendant ou après l'application de la composition réductrice à l'étape b et la tension est relâchée avant l'application de la composition à l'étape e.

**2.** Procédé selon la revendication 1, dans lequel la composition D comprend le premier acide (i) et le second acide (ii) dans un rapport en poids (i)/(ii) compris entre 10:1 et 1:250, de préférence entre 5:1 et 1:150, et plus préférentiellement entre 2:1 et 1:100, et de préférence encore entre 1:50.

**3.** Procédé selon les revendications 1 à 2, dans lequel la composition D est une poudre, une dispersion, une émulsion ou une solution, de préférence une composition aqueuse.

**4.** Procédé selon l'une quelconque des revendications précédentes, **caractérisé par le fait que** le pH de la composition D est compris entre 1 et 5, et que la composition D comprend un agent alcalinisant.

**5.** Procédé selon l'une quelconque des revendications précédentes, dans lequel l'acide carboxylique à trois groupes carboxyles ou plus est l'EDTA et/ou ses sels.

**6.** Procédé selon l'une quelconque des revendications précédentes, dans lequel l'acide organique à deux groupes carboxyles est l'acide malique et/ou ses sels.

**7.** Procédé selon l'une quelconque des revendications précédentes, dans lequel la composition B comprend un ou plusieurs précurseurs de colorants oxydants, de préférence choisis parmi les p-phénylendiamines, les p-aminophénols et les diamines hétérocycliques telles que les diaminepyrazols, et éventuellement une ou plusieurs substances de couplage de préférence choisies parmi les résorcinols, les m-aminophénols, les m-phénylendiamines, les pyridines et leurs dérivés, et les naphtols.

**8.** Procédé selon l'une quelconque des revendications précédentes, dans lequel la ou les compositions B et/ou D comprennent un ou plusieurs colorants capillaires directs choisis parmi les colorants cationiques, anioniques et nitrés ou leurs mélanges.

9. Procédé selon l'une quelconque des revendications précédentes, dans lequel la ou les compositions A et/ou B et/ou D comprennent au moins un agent alcalinisant choisi parmi l'ammoniaque, les alkylamines ou les alcanolamines, selon la structure générale suivante

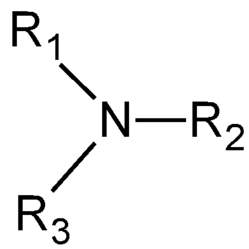

dans laquelle $R_1$, $R_2$ et $R_3$ sont identiques ou différents de H, de $C_1$ à $C_4$, d'alkyle insaturé de $C_3$ à $C_4$, d'alkyle ramifié de $C_3$ à $C_4$, d'alkyle hydroxyle de $C_1$ à $C_4$, d'alkyle hydroxyle insaturé de $C_3$ à $C_4$, d'alkyle hydroxyle ramifié de $C_3$ à $C_4$, à condition qu'au moins un des $R_1$, $R_2$ ou $R_3$ soit différent de H, les agents alcalinisants étant de préférence choisis parmi l'ammoniaque, la monoéthanolamine et l'aminométhylpropanol, l'aminométhylpropanol convenant particulièrement bien.

10. Procédé selon l'une quelconque des revendications précédentes, dans lequel la composition A, B, C et/ou D comprend un ou plusieurs ingrédients choisis parmi les alcools gras, les tensioactifs choisis parmi les tensioactifs anioniques, non ioniques, cationiques et amphotères, les ubiquinones, les céramides, les agents réducteurs, les solvants organiques, les silicones telles que les polysiloxanes linéaires, les silicones aminées, les silicones cycliques, les silicones arylées, les antioxydants, les conservateurs, les acides aminés, les polyols.

11. Procédé selon l'une quelconque des revendications précédentes, dans lequel la composition A et/ou B comprend des agents réducteurs choisis parmi l'acide thioglycolique, la cystéamine et/ou ses sels, la thioglycérine et/ou ses sels, les esters glycériques de l'acide thioglycolique et/ou ses sels, l'acide thiolactique et/ou ses sels, la cystéine ou ses dérivés et/ou ses sels, la cystéine ou ses dérivés et/ou ses sels, la cystéine ou ses dérivés et/ou ses sels, cystéine ou ses dérivés et/ou ses sels, dans laquelle la composition A comprend de préférence les agents réducteurs à une concentration allant de 1 % à 15 % en poids calculé par rapport au total de la composition A, et dans laquelle la composition B comprend de préférence les agents réducteurs à une concentration allant de 0. 1 % à 1,0 % en poids calculé par rapport au total de la composition B.

12. Procédé selon l'une quelconque des revendications précédentes, dans lequel la composition D comprend un ou plusieurs polymères épaississants choisis parmi les polymères anioniques, non ioniques, cationiques et amphotères, de préférence choisis parmi les polymères ayant une viscosité d'au moins 500 mPa-s mesurée à une concentration de polymère de 1 % en poids dans l'eau et à 20°C avec un viscosimètre Brookfield, par exemple à 10 tr/min pendant 1 minute, avec une broche appropriée.

13. Procédé selon la revendication 12, dans lequel le polymère épaississant est choisi parmi la gomme hydroxypropyl xanthane, la gomme déshydroxanthane, la gomme xanthane et les épaississants anioniques polymères.

14. Procédé selon l'une quelconque des revendications précédentes, dans lequel l'agent alcalinisant est compris dans la composition A, B et/ou D à une concentration comprise entre 0,1 % et 20 % en poids calculé par rapport au total de chaque composition.

15. Kit pour cheveux comprenant les compositions A, B, C et D telles que décrites dans les revendications 1 à 14.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1655056 A
- EP 2020254 A
- EP 2020255 A
- EP 2191865 A
- EP 2191864 A
- US 20150034119 A
- US 20150037270 A
- WO 2015017768 A
- WO 9515144 A